# EUROPEAN PATENT SPECIFICATION

(11) **EP 1 628 682 B1**
(45) Date of publication and mention of the grant of the patent: **25.09.2013**
(21) Application number: 04766017.0
(22) Date of filing: 19.05.2004
(51) Int. Cl.: A61K 31/505, A61K 31/205, A61K 31/44, A61K 31/522, A61K 31/53, A61P 11/00

(54) **COMPOSITION COMPRISING A PDE4 INHIBITOR AND A PDE5 INHIBITOR FOR THE TREATMENT OF COPD**
ZUSAMMENSETZUNG AUS EINEM PDE4 HEMMER UND EINEM PDE5 HEMMER ZUR BEHANDLUNG VON COPD
COMPOSITION CONTENANT UN INHIBITEUR DE PDE4 ET UN INHIBITEUR DE PDE5 POUR LE TRAITEMENT DE COPD

(30) Priority: 22.05.2003 EP 03011609
(43) Date of publication of application: 01.03.2006
(73) Proprietor: Takeda GmbH, 78467 Konstanz (DE)
(72) Inventor: DUNKERN, Thorsten, 41363 Jüchen Gierath (DE); HATZELMANN, Armin, 78467 Konstanz (DE); SCHUDT, Christian, 78462 Konstanz (DE); GRIMMINGER, Friedrich, 35510 Butzbach (DE); GHOFRANI, Hossein Ardeschir, 35398 Giessen (DE)
(74) Representative: Wild, Robert
(86) International application number: PCT/EP2004/050869
(87) International publication number: WO 2004/103407

(56) References cited:
- WO-A-02/064584
- COMPTON C H ET AL: "Cilomilast, a selective phosphodiesterase-4 inhibitor for treatment of patients with chronic obstructive pulmonary disease: a randomised, dose-ranging study" LANCET, XX, XX, vol. 358, no. 9278, 28 July 2001 (2001-07-28), pages 265-270, XP004297349 ISSN: 0140-6736
- MCPHERSON M A ET AL: "A cyclic nucleotide PDE5 inhibitor corrects defective mucin secretion in submandibular cells containing antibody directed against the cystic fibrosis transmembrane conductance regulator protein" FEBS LETTERS, ELSEVIER SCIENCE PUBLISHERS, AMSTERDAM, NL, vol. 464, no. 1-2, 24 December 1999 (1999-12-24), pages 48-52, XP004260719 ISSN: 0014-5793
- GHOFRANI H A ET AL: "Sildenafil for treatment of lung fibrosis and pulmonary hypertension: a randomised controlled trial" LANCET, XX, XX, vol. 360, no. 9337, 21 September 2002 (2002-09-21), pages 895-900, XP004381834 ISSN: 0140-6736
- WOLDA S L: "PDE4 INHIBITORS AND CHRONIC OBSTRUCTIVE PULMONARY DISEASE" EMERGING DRUGS, ASHLEY PUBLICATIONS, LONDON, GB, vol. 5, no. 3, 2000, pages 309-319, XP008016335 ISSN: 1361-9195
- BAYES M ET AL: "GATEWAYS TO CLINICAL TRIALS" METHODS AND FINDINGS IN EXPERIMENTAL AND CLINICAL PHARMACOLOGY, PROUS, BARCELONA, ES, vol. 24, no. 8, October 2002 (2002-10), pages 525-532, XP008020768 ISSN: 0379-0355
- TORPHY T J ET AL: "Phosphodiesterases: the journey towards therapeutics" TRENDS IN PHARMACOLOGICAL SCIENCES, ELSEVIER TRENDS JOURNAL, CAMBRIDGE, GB, vol. 21, no. 5, May 2000 (2000-05), pages 157-159, XP004198176 ISSN: 0165-6147
- DAL PIAZ V ET AL: "Phosphodiesterase 4 inhibitors, structurally unrelated to Rolipram, as promising agents for the treatment of asthma and other pathologies" EUROPEAN JOURNAL OF MEDICINAL CHEMISTRY, EDITIONS SCIENTIFIQUE ELSEVIER, PARIS, FR, vol. 35, no. 5, May 2000 (2000-05), pages 463-480, XP004330440 ISSN: 0223-5234

## Description

### Field of application of the invention

The invention relates to the combination of certain known active compounds for therapeutic purposes. The substances used in the combination according to the invention are known active compounds from the phosphodiesterase 4 (PDE4) inhibitor class and known active compounds from the phosphodiesterase 5 (PDE5) inhibitor class. Their combined use in the sense according to the invention for therapeutic purposes has not yet been described in prior art.

### Prior art

In the healthy lung of humans both at rest and during exercise there are always areas of good and poor or absolutely no ventilation existing simultaneously side by side (ventilation inhomogeneity). An as yet unknown mechanism ensures that there is little or no perfusion of the capillaries adjacent to alveoli with little or no ventilation. This occurs in order to minimize inefficient perfusion of areas of the lung which are not involved in gas exchange. During bodily exercise, the distribution of ventilation changes (recruitment of new alveoli) and there is increased perfusion of the relevant capillary bed. Conversely, when there is less ventilation due to physiological or pathological processes (airway obstruction), the capillary flow is reduced through vasoconstriction. This process is referred to as "hypoxic vasoconstriction" (Euler-Liljestrand mechanism). When this adaptation mechanism is impaired ("mismatch"), there may, despite adequate ventilation and normal perfusion of the lungs, be a more or less pronounced collapse of the gas exchange function, which can be compensated only inadequately despite a further increase in ventilation or perfusion. Under these conditions there are regions which are not ventilated but are well perfused (shunt flow perfusion) and those which are well ventilated but not perfused (dead space ventilation). The consequences of this ventilation/perfusion mismatch are hypoxaemia (deterioration in gas exchange with an associated decrease in the arterial oxygen saturation), wasted perfusion (uneconomical perfusion of unventilated areas) and wasted ventilation (uneconomical ventilation of poorly perfused areas).

In patients with inflammatory and degenerative lung disorders such as, for example, chronic obstructive pulmonary disease (COPD), bronchitis, bronchial asthma, pulmonary fibroses, emphysema, interstitial pulmonary disorders and pneumonias there is observed to be partial or global respiratory failure. The cause is inadequate adaptation of the intrapulmonary perfusion conditions to the inhomogeneous pattern of the distribution of ventilation. The mismatch derives from the effect of vasoactive (inflammatory) mediators which prevail over the physiological adaptation mechanism and in part from structural changes of the lung capillaries which develop during disease progression. This effect is particularly evident during exercise and when the oxygen demand is increased and it is manifested by dyspnoea (hypoxia) and limitation of performance.

Chronic Obstructive Pulmonary Disease (COPD) is a major public health problem projected to rank fifth in 2020 as a worldwide burden of disease according to a study published by the World Bank/World Health Organization [Murray CJL, Lopez AD. Evidence-based health policy - lessons from the global burden of disease study. Science 1996; 274:740-3]. COPD is a disease state characterized by airflow limitation that is not fully reversible, also upon treatment with bronchodilators. The airflow limitation is usually both progressive and associated with an abnormal inflammatory response of the lungs to noxious particles or gases (e.g. cigarette smoke). COPD is characterized by chronic inflammation throughout the airways, parenchyma, and pulmonary vasculature. This inflammation mechanistically clearly differs from that of asthma, which might explain the limited benefit of corticosteroid treatment in stable disease management of those patients. In addition, other processes are thought to be important in the pathogenesis of COPD, i.e. structural changes/remodelling of the airways as well as of the pulmonary capillaries leading to reduced blood perfusion and an endothelial dysfunction. Up to now a curative therapy for COPD is not available. In use are anti-cholinergic drugs (ipratropium bromide, tiotropium bromide and oxitropium bromide) and short- and long-acting β-adrenoreceptor-agonists (salmeterol, terbutalin-sulphate). β2-agonist-treatment can be associated with several side-effects such as tachycardia, unrest felling, sleep disturbances and tremor. Observed side-effects of tiotropium bromide and ipratropium bromide treatment are the development of a dry mouth, tremor, tachykardia and obstipation. Anti-inflammatory acting corticosteroids are not fully established for COPD treatment, since their effects in the daily management of stable COPD are very small, provide only symptomatic relief, do not improve survival and side-effects (e.g. development of sooroesophagitis) have to be considered.

The 3', 5'-cyclic nucleotide phosphodiesterases (PDEs) comprise a large class of enzymes divided into at least eleven different families which are structurally, biochemically and pharmacologically distinct from one another.

PDE4s are characterized by selective, high affinity hydrolytic degradation of the second messenger cyclic nucleotide, adenosine 3', 5'-cyclic monophosphate (cAMP). A number of selective and potent inhibitors of the PDE4s have been discovered in recent years for treatment of COPD or Asthma.

Known PDE4 inhibitors are reviewed in Dal Piaz et al. [Dal Piaz V et al. (2000) European Journal of Medicinal Chemistry 35: 463] and Wolda [Wolda SL. PDE4 Inhibitors and chronic obstructive pulmnonary disease. Emerging Drugs, Ashley Publications, London, GB, Vol. 5, No. 3, 2000, pages 309-319].

Compton et al. (Comptom CH et al. (2001) Lancet 358: 265) discloses the effectiveness of maintenance treatment of COPD patients with the PDE4 inhibitor Cilomilast.

PDE5s are characterized by selective, high affinity hydrolytic degradation of the second messenger cyclic nucleotide, guanosine 3', 5'-cyclic monophosphate (cGMP). A whole series of PDE5 inhibiting substances are known from the prior art and are described as potent and effective substances for the treatment of erectile dysfunction and pulmonary hypertension.

Ghofrani et al. (Ghofrani HA et al. (2002) Lancet 360: 895) disclose effects of a Sildenafil therapy on patients with lung fibrosis and pulmonary hypertension. Ghofrani et al. demonstrates that Sildenafil acts selectively in well-ventilated areas of the lung by improving the gas exchange.

McPhershon et al. (McPhershon et al. (1999) FEBS Letters 464: 48) demonstrate that a PDE5-inhibitor restores the defect in isoproterenol induced mucine secretion in cells, which resemble the phenotype of cells involved in cystic fibrosis.

Torphy et al. (Torphy TJ et al. (2000) Trends in Pharmacological Sciences 21: 157) discloses in a meeting report that the action of PDE4-inhibitors in COPD and RA is due to their well-known suppression of the release of inflammatory mediators, as TNF and IL-12 from various inflammatory cells (neutrophils, macrophages). In the same report, Torphy et al. describes the use of PDE5-inhibitors for the treatment of erectile dysfunction.

### Description of the invention

The invention relates to pharmaceutical compositions comprising a PDE4 inhibitor in combination with a PDE5 inhibitor and the use of this combination for the preparation of a medicament for preventing or reducing the onset of symptoms of COPD, or treating or reducing the severity of COPD.

In particular it relates to compositions and the use thereof for treating a disease mediated by phosphodiesterase 4 (PDE4) and/or phosphodiesterase 5 (PDE5) activity by administering a PDE4 inhibitor in combination with a PDE5 inhibitor.

In this connection, it is the object of the present invention to make available a certain therapeutic, which fulfills the following conditions:
- Pronounced anti-inflammatory action and
- Rematching effect and/or anti-remodelling effect.

According to present invention, the term "rematching effect" refers to the ability of the combined use of a PDE4 inhibitor and a PDE5 inhibitor to dilate vessels in the pulmonary circulation and, at the same time, to redistribute the blood flow within the lung in favour of the well-ventilated areas of the lung. Thereby the disease associated shunt perfusion within the lung is reduced. Rematching leads to an improvement in the gas exchange function both at rest and during physical exercise and thereby to an improvement in arterial oxygen saturation.

According to present invention, the term "anti-remodelling effect" refers to the ability of the combined use of a PDE4 inhibitor and a PDE5 inhibitor to restore the impaired balance between proliferation and cell death of smooth muscle cells, fibroblasts and epithelial cells and/or to reduce excessive production of extracellular matrix in the vasculature of the lung.

It has now been found that the combined use of a PDE4 inhibitor and a PDE5 inhibitor fulfills the above mentioned conditions.

Accordingly, in a first aspect the invention relates to the use of a combination of a PDE4 inhibitor and a PDE5 inhibitor for the preparation of a medicament for preventing or reducing the onset of symptoms of COPD, or treating or reducing the severity of COPD , wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In a second aspect the present invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione(TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the first aspect, the invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

In a further embodiment of the first aspect, the invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the first aspect, the invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In an embodiment of the second aspect, the invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

In a further embodiment of the second aspect, the invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the second aspect, the invention relates to the use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 5-(2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In a third aspect the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the third aspect, the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

In a further embodiment of the third aspect, the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the third aspect, the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In a fourth aspect the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the fourth aspect, the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]-pyrido[3,4-b]indole-1,4-dione (TADALAFIL) or a pharmaceutically acceptable salt thereof.

In a further embodiment of the fourth aspect, the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the fourth aspect, the invention relates to a pharmaceutical composition comprising as a fixed combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In a fifth aspect the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo(4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the fifth aspect, the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

In a further embodiment of the fifth aspect, the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the fifth aspect, the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In a sixth aspect the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the sixth aspect, the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]-pyrido[3,4-b]indole-1,4-dione (TADALAFIL) or a pharmaceutically acceptable salt thereof.

In a further embodiment of the sixth aspect, the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the sixth aspect, the invention relates to a pharmaceutical composition comprising as a fixed oral combination (a) an effective amount of a PDE4 inhibitor, (b) an effective amount of a PDE5 inhibitor, and optionally (c) a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In a seventh aspect the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the seventh aspect, the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

In a further embodiment of the seventh aspect, the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the seventh aspect, the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

In a eighth aspect the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

In an embodiment of the eighth aspect, the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]-pyrido[3,4-b]indole-1,4-dione (TADALAFIL) or a pharmaceutically acceptable salt thereof.

In a further embodiment of the eighth aspect, the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

In a further embodiment of the eighth aspect, the invention relates to a pharmaceutical composition comprising as a free combination (a) an effective amount of a PDE4 inhibitor and optionally a pharmaceutically acceptable carrier and (b) an effective amount of a PDE5 inhibitor and optionally a pharmaceutically acceptable carrier, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

The combination therapy, which is the subject matter of present invention, comprises administering a PDE4 inhibitor with a PDE5 inhibitor to prevent the onset of COPD.

The invention thus relates to the combined use of a PDE4 inhibitor and a PDE5 inhibitor in preventing the symptoms of, or treating COPD.

PDE4 inhibitors that may be usefully employed in the present invention include a compound selected from 3-Cyclopropylmethoxy-4-difluoromethoxy-N-(3,5-dichloropyrid-4-yl)-benzamide [INN: ROFLUMILAST] as well as its N-oxide [3-cyclopropylmethoxy-4-difluoromethoxy-N-(3,5-dichloro-1-oxypyrid-4-yl)-benzamide and the pharmaceutically acceptable salts of these compounds.

The preparation of these compounds and and their pharmaceutically acceptable salts as well as their use as PDE4 inhibitors is disclosed in the international patent application WO95/01338.

PDE5 inhibitors that may be usefully employed in the present invention include a compound selected from
- TADALAFIL: (6R,12aR)-2,3,6,7,12,12a-hexahydro-2-methyl-6-(3,4-methylenedioxy-phenyl)-pyrazino[2',1':6,1]pyrido[3,4-b]indole-1,4-dione;
- VARDENAFIL: 2-[2-ethoxy-5-(4-ethylpiperazin-1-ylsulfonyl)phenyl]-5-methyl-7-propylimidazo[5,1-f][1,2,4]triazin-4(3H)-one;
- SILDENAFIL: 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one;
and the pharmaceutically acceptable salts of these compounds.

The preparation of TADALAFIL and its pharmaceutically acceptable salts as well as their use as PDE5 inhibitors is disclosed in the patent application WO9519978. The preparation of VARDENAFIL and its pharmaceutically acceptable salts as well as their use as PDE5 inhibitors is disclosed in the patent application WO9924433. The preparation of SILDENAFIL and its pharmaceutically acceptable salts as well as their use as PDE5 inhibitors is disclosed in the patent application EP0463756.

The phrase "combined use" (or "combination") embraces the administration of a PDE4 inhibitor and a PDE5 inhibitor as part of a specific treatment regimen intended to provide a beneficial effect from the co-action of these therapeutic agents. Administration of these therapeutic agents in combination typically is carried out over a defined time period (usually minutes, hours, days or weeks depending upon the combination selected). "Combined use" generally is not intended to encompass the administration of two of these therapeutic agents as part of separate monotherapy regimens that incidentally and arbitrarily result in the combinations of the present invention.

"Combined use" or "combination" within the meaning of the present invention is to be understood as meaning that the individual components can be administered simultaneously (in the form of a combination medicament - "fixed combination") or more or less simultaneously, or respectively in succession (from separate pack units - "free combination"; directly in succession or else alternatively at a relatively large time interval). As an example, one therapeutic agent could be taken in the morning and one later in the day. Or in another scenario, one therapeutic agent could be taken once daily and the other twice weekly. It is understood, that if individual components are administered directly in succession, the delay in administering the second component should not be such as to lose the beneficial therapeutic effect of the combination.

Simultaneous administration can be effected by any appropriate route and, preferably, is accomplished, for example, by administering the therapeutic agents to the subject in need thereof by the oral route, or the intravenous route, or the intramuscular route, or by subcutaneous injection whereby the administration form has a fixed ratio of each therapeutic agent.

More or less simultaneous administration or administration in succession of each therapeutic agent can be effected by any appropriate route, including, but not limited to, oral routes, intravenous routes, intramuscular routes, and direct absorption through mucous membrane tissues. The therapeutic agents can be administered by the same route or by different routes. For example, both therapeutic agents of the combination may be administered by orally. In another example, a first therapeutic agent of the combination selected may be administered by intravenous or subcutaneous injection while the other therapeutic agent of the combination may be administered orally. The sequence in which the therapeutic agents are administered is not narrowly critical.

The most preferred route of administration of Roflumilast, a pharmaceutically acceptable salt of Roflumilast, Roflumilast-N-oxide or a pharmaceutically acceptable salt of Roflumilast-N-oxide is the oral route. In another preferred embodiment Roflumilast, a pharmaceutically acceptable salt of Roflumilast, Roflumilast-N-oxide or a pharmaceutically acceptable salt of Roflumilast-N-oxide is administered by intravenous infusion or injection. In a further embodiment Roflumilast, a pharmaceutically acceptable salt of Roflumilast, Roflumilast-N-oxide or a pharmaceutically acceptable salt of Roflumilast-N-oxide is administered by intramuscular or subcutaneous injection. Other routes of administration are also contemplated, including intranasal and transdermal routes, and by inhalation.

The most preferred route of administration of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) or a pharmaceutically acceptable salt of these compounds is the oral route. In another preferred embodiment 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) or a pharmaceutically acceptable salt of these compounds is administered by intravenous infusion or injection. In a further embodiment 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) or a pharmaceutically acceptable salt of these compounds is administered by intramuscular or subcutaneous injection. Other routes of administration are also contemplated, including intranasal and transdermal routes, and by inhalation.

The therapeutic agent(s) of the present invention can be administered by a variety of methods known in the art, although for many therapeutic applications, the preferred route of administration for a fixed combination of a PDE4 inhibitor and a PDE5 inhibitor according to the invention is the oral route. The preferred route of administration for a free combination of a PDE4 inhibitor and a PDE5 inhibitor according to present invention is the oral route.

In case of pharmaceutical compositions, which are intended for oral administration, the therapeutic agent(s) are formulated to give medicaments according to processes known per se and familiar to the person skilled in the art. The therapeutic agents are employed as medicament, preferably in combination with suitable pharmaceutical carrier, in the form of tablets, coated tablets, capsules, caplets, emulsions, suspensions, syrups or solutions, the therapeutic agent content advantageously being between 0.1 and 95% by weight and, by the appropriate choice of the carrier, it being possible to achieve a pharmaceutical administration form precisely tailored to the therapeutic agent(s) and/or to the desired onset of action (e.g. a sustained-release form or an enteric form).

The person skilled in the art is familiar on the basis of his/her expert knowledge which carriers or excipients are suitable for the desired pharmaceutical formulations. In addition to solvents, gel-forming agents, tablet excipients and other active compound carriers, it is possible to use, for example, antioxidants, dispersants, emulsifiers, antifoams, flavor corrigents, preservatives, solubilizers, colorants or permeation promoters and complexing agents (e.g. cyclodextrins).

Suitable oral dosage forms of Roflumilast are described in the international patent application WO03/070279.

The therapeutic agent(s) are dosed in an order of magnitude customary for the individual dose. It is more likely possible that the individual actions of the therapeutic agents are mutually positively influenced and reinforced and thus the respective doses on the combined administration of the therapeutic agent(s) may be reduced compared with the norm.

In case of oral, intravenous or subcutaneous administration of a PDE4 inhibitor, the daily dose will likely be in the range from 0.001 to 3 mg/kg body weight of the subject to be treated, preferably by once daily administration.

In case of oral administration of 3-cyclopropylmethoxy-4-difluoromethoxy-N-(3,5-dichloropyrid-4-yl)-benzamide (ROFLUMILAST), the adult daily dose is in the range from 50 - 1000 µg, preferably in the range from 250 - 500µg, preferably by once daily administration.

In case of oral, intravenous or subcutaneous administration of a PDE5 inhibitor, the daily dose will likely be in the range from 0.001 to 3 mg/kg body weight of the subject to be treated, preferably by once daily administration.

Tablet formulations for sildenafil, tadalafil and vardenafil are commercially available under the tradenames Viagra®, Cialis® and Levitra® respectively.

Commercially available tablet formulations for sildenafil contain 25, 50 or 100 mg of sildenafil. According to the Summary of Product Characteristics for Sildenafil, as a monotherapy the PDE5 inhibitor Sildenafil is generally administered orally to adults in a daily dose of 25, 50 or 100 mg.

Commercially available tablet formulations for vardenafil contain 5, 10 or 20 mg of vardenafil. According to the Summary of Product Characteristics for Vardenafil, as a monotherapy the PDE5 inhibitor Vardenafil is generally administered orally to adults in a daily dose of 5, 10 or 20 mg.

Commercially available tablet formulations for tadalafil contain 10 or 20 mg of tadalafil. According to the Summary of Product Characteristics for Tadalafil, as a monotherapy the PDE5 inhibitor Tadalafil is generally administered orally to adults in a daily dose of 10 or 20 mg.

### Examples

### Administration of the Combination

### Example 1

Patients suffering from COPD as defined by the "Global Initiative for chronic obstructive lung disease" (Pauwels R.A., et al., Global strategy for the diagnosis, management, and prevention of chronic obstructive pulmonary disease. NHLBI/WHO Global Initiative for Chronic Obstructive Lung Disease (GOLD) Workshop summary. Am. J. Respir. Crit. Care Med. 2001; 163: 1256-1276) are administered orally one tablet of Roflumilast (comprising 500 µg of Roflumilast) per day and once daily a tablet of Viagra (comprising 50 mg Sildenafil).

### Example 2

Patients suffering from COPD as defined by the "Global Initiative for chronic obstructive lung disease" (Pauwels R.A., et al., Global strategy for the diagnosis, management, and prevention of chronic obstructive pulmonary disease. NHLBI/WHO Global Initiative for Chronic Obstructive Lung Disease (GOLD) Workshop summary. Am. J. Respir. Crit. Care Med. 2001; 163: 1256-1276) are administered orally one tablet of Roflumilast (comprising 500 µg of Roflumilast) per day and each second day a tablet of Cialis (comprising 10 mg Tadalafil).

### Example 3

Patients suffering from COPD as defined by the "Global Initiative for chronic obstructive lung disease" (Pauwels R.A., et al., Global strategy for the diagnosis, management, and prevention of chronic obstructive pulmonary disease. NHLBI/WHO Global Initiative for Chronic Obstructive Lung Disease (GOLD) Workshop summary. Am. J. Respir. Crit. Care Med. 2001; 163: 1256-1276) are administered orally one tablet of Roflumilast (comprising 500 µg of Roflumilast) per day and once daily a tablet of Levitra (comprising 10 mg Vardenafil).

### Utility

Because of their PDE4- and PDE5- inhibitory properties, combinations of present invention are applicable in human and veterinary medicine, wherein - as an example - the combinations useful for preventing or reducing the onset of symptoms of a disease, or treating or reducing the severity of a disease in a patient in need thereof, in which disease phosphodiesterase 4 (PDE4) and/or phosphodiesterase 5 (PDE5) activity is detrimental. Said diseases are of varying origin and are characterized by an inflammatory component, a mismatch component and structural changes in the morphology of the airways (remodelling component). Thus, the combined use of a PDE4 inhibitor and a PDE5 inhibitor in accordance with present invention is applicable for preventing or reducing the onset of inflammation, or treating or reducing the severity of inflammation, mismatch and remodelling.

Surprisingly it has been found that the combined use of a PDE4- and a PDE5-inhibitor in the treatment of COPD is superior to a treatment with either a PDE4-inhibitor or a PDE5-inhibitor as the combined use of a PDE4- and a PDE5-inhibitor lead to a synergistic effect. This synergistic effect refers to an intensive mechanistic crosstalk between the pathomechanisms influenced by PDE4-inhibitors and those of PDE5-inhibitors. For example immune cells involved in COPD, which activity can be suppressed by treatment with PDE4-inhibitors, may release cytokines and growth factors, which induce and influence structural remodelling processes of vasculature. These remodelling processes are also under the control of PDE5-inhibitors, which are known to influence proliferation. Thus, combined use of a PDE4- and PDE5-inhibitor for the treatment of COPD is more effective than treatment with the individual inhibitors. In addition, treatment of COPD by use of a composition comprising a PDE4- and a PDE5-inhibitor allows the adaptation of a dose scheme of both inhibitors in order to get a useful ratio of plasma concentrations of the PDE4- and PDE5-inhibitor by considering the different pharmacokinetic behaviour of these drugs.

## Claims

1. Use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

2. Use of a combination of a PDE4 inhibitor and a PDE5 inhibitor for the preparation of a medicament for preventing or reducing the onset of symptoms of COPD, or treating or reducing the severity of COPD, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

3. Use according to claim 1, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

4. Use according to claim 1, wherein the PDE4 inhibitor is 3-cyclopropylmethoxy-4-difluoromethoxy-N-(3,5-dichloropyrid-4-yl)benzamide (Roflumilast) or a pharmaceutically
acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

5. Use according to claim 1, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

6. Use according to claim 2, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

7. Use according to claim 2, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

8. Use according to claim 2, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

9. Pharmaceutical composition comprising as a fixed combination
(a) an effective amount of a PDE4 inhibitor and
(b) an effective amount of a PDE5 inhibitor, and optionally
(c) a pharmaceutically acceptable carrier
wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 6-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL),. (6R,12aR)-2,3,6,7,12,12a-hexahydro-2-methyl-6-(3,4-methylenedioxyphenyl)-pyrazino[2',1':6,1]pyrido(3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

10. Pharmaceutical composition comprising as a fixed oral combination
(a) an effective amount of a PDE4 inhibitor and
(b) an effective amount of a PDE5 inhibitor, and optionally
(c) a pharmaceutically acceptable carrier
wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-l-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

11. Pharmaceutical composition comprising as a free combination
(a) an effective amount of a PDE4 inhibitor and optionally a pharmaceutically acceptable carrier and
(b) an effective amount of a PDE5 inhibitor and optionally a pharmaceutically acceptable carrier
wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

12. Pharmaceutical composition comprising as a fixed combination
(a) an effective amount of a PDE4 inhibitor and
(b) an effective amount of a PDE5 inhibitor, and optionally
(c) a pharmaceutically acceptable carrier
wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

13. Pharmaceutical composition comprising as a fixed oral combination
(a) an effective amount of a PDE4 inhibitor and
(b) an effective amount of a PDE5 inhibitor, and optionally
(c) a pharmaceutically acceptable carrier
wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

14. Pharmaceutical composition comprising as a free combination
(a) an effective amount of a PDE4 inhibitor and optionally a pharmaceutically acceptable carrier and
(b) an effective amount of a PDE5 inhibitor and optionally a pharmaceutically acceptable carrier
wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is selected from the group consisting of 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL), (6R,12aR)-2,3,6,7,12,12a-hexahydro-2-methyl-6-(3,4-methylenedioxyphenyl)-pyrazino[2',1':6,1]pyrido[3,4-b]indole-1,4-dione (TADALAFIL), 2-[2-ethoxy-5-(4-ethylpiperazin-1-ylsulfonyl)phenyl]-5-methyl-7-propylimidazo[5,1-f][1,2,4]triazin-4(3H)one (VARDENAFIL) and the pharmaceutically acceptable salts of these compounds.

15. Pharmaceutical composition according to any of claims 9 to 11, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]pyrido[3,4-b]indole-1,4-dione (TADALAFIL) or a pharmaceutically acceptable salt thereof.

16. Pharmaceutical composition according to any of claims 9 to 11, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2.4]triazin-4(3H)-one (VARDENAFIL) or a pharmaceutically acceptable salt thereof.

17. Pharmaceutical composition according to any of claims 9 to 11, wherein the PDE4 inhibitor is 3-cyclopropylmethoxy-4-difluoromethoxy-N-(3,5-dichloropyrid-4-yl)benzamide (Roflumilast) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

18. Pharmaceutical composition according to any of claims 12 to 14, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is (6R,12aR)-2,3,6,7,12,12a-hexahydro-2-methyl-6-(3,4-methylenedioxy-phenyl)-pyrazino[2',1':6,1]-pyrido[3,4-b]indole-1,4-dione (TADALAFIL) or a pharmaceutically acceptable salt thereof.

19. Pharmaceutical composition according to any of claims 12 to 14, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 2-[2-ethoxy-5-(4-ethylpiperazin-1-ylsulfonyl)phenyl]-5-methyl-7-propylimidazo[5,1-f][1,2,4]triazin-4(3H)-one (VARDENAFIL) or a pharmaceutically acceptable salt thereof.

20. Pharmaceutical composition according to any of claims 12 to 14, wherein the PDE4 inhibitor is 3-cyclopropylmethoxy-4-difluoromethoxy-N-(3,5-dichloro-1-oxy-pyrid-4-yl)benzamide (Roflumilast-N-oxide) or a pharmaceutically acceptable salt thereof and the PDE5 inhibitor is 5-[2-ethoxy-5-(4-methyl-1-piperazinylsulfonyl)phenyl]-1-methyl-3-n-propyl-1,6-dihydro-7H-pyrazolo[4,3-d]pyrimidin-7-one (SILDENAFIL) or a pharmaceutically acceptable salt thereof.

## Patentansprüche

1. Verwendung einer Kombination eines PDE4-Hemmers und eines PDE5-Hemmers für die Herstellung eines Arzneimittels zur Vorbeugung oder zum Reduzieren des Ausbruchs von COPD-Symptomen, oder zum Behandeln oder Reduzieren der Schwere von COPD, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on **(SILDENAFIL),** (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

2. Verwendung einer Kombination eines PDE4-Hemmers und eines PDE5-Hemmers für die Herstellung eines Arzneimittels zur Vorbeugung oder zum Reduzieren des Ausbruchs von COPD-Symptomen, oder zum Behandeln oder Reduzieren der Schwere von COPD, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

3. Verwendung nach Anspruch 1, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt und es sich bei dem PDE5-Hemmer um (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL) oder ein pharmazeutisch unbedenkliches Salz davon handelt.

4. Verwendung nach Anspruch 1, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt und es sich bei dem PDE5-Hemmer um 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) oder ein pharmazeutisch unbedenkliches Salz davon handelt.

5. Verwendung nach Anspruch 1, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt und es sich bei dem PDE5-Hemmer um 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL) oder ein pharmazeutisch unbedenkliches Salz davon handelt.

6. Verwendung nach Anspruch 2, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt und es sich bei dem PDE5-Hemmer um (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL) oder ein pharmazeutisch unbedenkliches Salz davon handelt.

7. Verwendung nach Anspruch 2, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt und es sich bei dem PDE5-Hemmer um 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) oder ein pharmazeutisch unbedenkliches Salz davon handelt.

8. Verwendung nach Anspruch 2, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt und es sich bei dem PDE5-Hemmer um 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL) oder ein pharmazeutisch unbedenkliches Salz davon handelt.

9. Pharmazeutische Zusammensetzung, die als feststehende Kombination Folgendes umfasst:
(a) eine wirksame Menge eines PDE4-Hemmers und
(b) eine wirksame Menge eines PDE5-Hemmers und gegebenenfalls
(c) einen pharmazeutisch unbedenklichen Träger,
wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

10. Pharmazeutische Zusammensetzung, die als feststehende orale Kombination Folgendes umfasst:
(a) eine wirksame Menge eines PDE4-Hemmers und
(b) eine wirksame Menge eines PDE5-Hemmers und gegebenenfalls
(c) einen pharmazeutisch unbedenklichen Träger,
wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

11. Pharmazeutische Zusammensetzung, die als freie Kombination Folgendes umfasst:
(a) eine wirksame Menge eines PDE4-Hemmers und gegebenenfalls einen pharmazeutisch unbedenklichen Träger und
(b) eine wirksame Menge eines PDE5-Hemmers und gegebenenfalls einen pharmazeutisch unbedenklichen Träger,
wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

12. Pharmazeutische Zusammensetzung, die als feststehende Kombination Folgendes umfasst:
(a) eine wirksame Menge eines PDE4-Hemmers und
(b) eine wirksame Menge eines PDE5-Hemmers und gegebenenfalls
(c) einen pharmazeutisch unbedenklichen Träger,
wobei es sich bei dem PDE4-Hemmer um 3-Cyclopropylmethoxy-4-difluormethoxy-N-(3,5-dichlor-1-oxypyrid-4-yl)benzamid (Roflumilast-N-oxid) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

13. Pharmazeutische Zusammensetzung, die als feststehende orale Kombination Folgendes umfasst:
(a) eine wirksame Menge eines PDE4-Hemmers und
(b) eine wirksame Menge eines PDE5-Hemmers und gegebenenfalls
(c) einen pharmazeutisch unbedenklichen Träger,
wobei es sich bei dem PDE4-Hemmer um 3-Cyclopropylmethoxy-4-difluormethoxy-N-(3,5-dichlor-1-oxypyrid-4-yl)benzamid (Roflumilast-N-oxid) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

14. Pharmazeutische Zusammensetzung, die als freie Kombination Folgendes umfasst:
(a) eine wirksame Menge eines PDE4-Hemmers und gegebenenfalls einen pharmazeutisch unbedenklichen Träger und
(b) eine wirksame Menge eines PDE5-Hemmers und gegebenenfalls einen pharmazeutisch unbedenklichen Träger,
wobei es sich bei dem PDE4-Hemmer um 3-Cyclopropylmethoxy-4-difluormethoxy-N-(3,5-dichlor-1-oxypyrid-4-yl)benzamid (Roflumilast-N-oxid) oder ein pharmazeutisch unbedenkliches Salz davon handelt, und wobei der PDE5-Hemmer aus der Gruppe bestehend aus 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL), (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL), 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) und den pharmazeutisch unbedenklichen Salzen dieser Verbindungen ausgewählt ist.

15. Pharmazeutische Zusammensetzung nach einem der Ansprüche 9 bis 11, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt und es sich bei dem PDE5-Hemmer um (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL) oder ein pharmazeutisch unbedenkliches Salz davon handelt.

16. Pharmazeutische Zusammensetzung nach einem der Ansprüche 9 bis 11, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt und es sich bei dem PDE5-Hemmer um 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) oder ein pharmazeutisch unbedenkliches Salz davon handelt.

17. Pharmazeutische Zusammensetzung nach einem der Ansprüche 9 bis 11, wobei es sich bei dem PDE4-Hemmer um 3-Cyclopropylmethoxy-4-difluormethoxy-N-(3,5-dichlorpyrid-4-yl)benzamid (Roflumilast) oder ein pharmazeutisch unbedenkliches Salz davon handelt und es sich bei dem PDE5-Hemmer um 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL) oder ein pharmazeutisch unbedenkliches Salz davon handelt.

18. Pharmazeutische Zusammensetzung nach einem der Ansprüche 12 bis 14, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt und es sich bei dem PDE5-Hemmer um (6R,12aR)-2,3,6,7,12,12a-Hexahydro-2-methyl-6-(3,4-methylendioxyphenyl)pyrazino[2',1':6,1]pyrido[3,4-b]indol-1,4-dion (TADALAFIL) oder ein pharmazeutisch unbedenkliches Salz davon handelt.

19. Pharmazeutische Zusammensetzung nach einem der Ansprüche 12 bis 14, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt und es sich bei dem PDE5-Hemmer um 2-[2-Ethoxy-5-(4-ethylpiperazin-1-ylsulfonyl)phenyl]-5-methyl-7-propylimidazo[5,1-f][1,2,4]triazin-4(3H)on (VARDENAFIL) oder ein pharmazeutisch unbedenkliches Salz davon handelt.

20. Pharmazeutische Zusammensetzung nach einem der Ansprüche 12 bis 14, wobei es sich bei dem PDE4-Hemmer um 3-Cyclopropylmethoxy-4-difluormethoxy-N-(3,5-dichlor-1-oxypyrid-4-yl)benzamid (Roflumilast-N-Oxid) oder ein pharmazeutisch unbedenkliches Salz davon handelt und es sich bei dem PDE5-Hemmer um 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (SILDENAFIL) oder ein pharmazeutisch unbedenkliches Salz davon handelt.

## Revendications

1. Utilisation d'une combinaison d'un inhibiteur de la PDE4 et d'un inhibiteur de la PDE5 pour la préparation d'un médicament destiné à prévenir ou à réduire l'apparition de symptômes de la BPCO, ou traiter ou réduire la gravité de la BPCO, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est choisi dans le groupe constitu é par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

2. Utilisation d'une combinaison d'un inhibiteur de la PDE4 et d'un inhibiteur de la PDE5 pour la préparation d'un médicament destiné à prévenir ou à réduire l'apparition de symptômes de la BPCO, ou traiter ou réduire la gravité de la BPCO, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

3. Utilisation selon la revendication 1, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est la (6R,12aR)-2,3,6,7,12,12a-hexahydro-2-méthyl-6-(3,4-méthylènedioxy-phényl)-pyrazino[2',1':6,1]pyrido[3,4-b]indole-1,4-dione (TADALAFIL) ou un sel pharmaceutiquement acceptable de celle-ci.

4. Utilisation selon la revendication 1, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) ou un sel pharmaceutiquement acceptable de celle-ci.

5. Utilisation selon la revendication 1, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL) ou un sel pharmaceutiquement acceptable de celle-ci.

6. Utilisation selon la revendication 2, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est la (6R,12aR)-2,3,6,7,12,12a-hexahydro-2-méthyl-6-(3,4-méthylènedioxy-phényl)-pyrazino[2',1':6,1]pyrido[3,4-b]indole-1,4-dione (TADALAFIL) ou un sel pharmaceutiquement acceptable de celle-ci.

7. Utilisation selon la revendication 2, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) ou un sel pharmaceutiquement acceptable de celle-ci.

8. Utilisation selon la revendication 2, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL) ou un sel pharmaceutiquement acceptable de celle-ci.

9. Composition pharmaceutique comprenant en tant que combinaison fixe
(a) une quantité efficace d'un inhibiteur de la PDE4, et
(b) une quantité efficace d'un inhibiteur de la PDE5, et éventuellement
(c) un support pharmaceutiquement acceptable
**caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

10. Composition pharmaceutique comprenant en tant que combinaison orale fixe
(a) une quantité efficace d'un inhibiteur de la PDE4, et
(b) une quantité efficace d'un inhibiteur de la PDE5, et éventuellement
(c) un support pharmaceutiquement acceptable
**caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

11. Composition pharmaceutique comprenant en tant que combinaison libre
(a) une quantité efficace d'un inhibiteur de la PDE4 et éventuellement un support pharmaceutiquement acceptable, et
(b) une quantité efficace d'un inhibiteur de la PDE5 et éventuellement un support pharmaceutiquement acceptable
**caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

12. Composition pharmaceutique comprenant en tant que combinaison fixe
(a) une quantité efficace d'un inhibiteur de la PDE4, et
(b) une quantité efficace d'un inhibiteur de la PDE5, et éventuellement
(c) un support pharmaceutiquement acceptable
**caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

13. Composition pharmaceutique comprenant en tant que combinaison fixe orale
(a) une quantité efficace d'un inhibiteur de la PDE4, et
(b) une quantité efficace d'un inhibiteur de la PDE5, et éventuellement
(c) un support pharmaceutiquement acceptable
**caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

14. Composition pharmaceutique comprenant en tant que combinaison libre
(a) une quantité efficace d'un inhibiteur de la PDE4 et éventuellement un support pharmaceutiquement acceptable, et
(b) une quantité efficace d'un inhibiteur de la PDE5 et éventuellement un support pharmaceutiquement acceptable
**caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est choisi dans le groupe constitué par la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL), la (6R,12aR)-2,3,6,7,12,12a-hexahydro-2-méthyl-6-(3,4-méthylènedioxyphényl)-pyrazino[2',1':6,1]pyrido[3,4-b]indole-1,4-dione (TADALAFIL), la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) et les sels pharmaceutiquement acceptables de ces composés.

15. Composition pharmaceutique selon l'une quelconque des revendications 9 à 11, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est la (6R,12aR)-2,3,6,7,12,12a-hexahydro-2-méthyl-6-(3,4-méthylènedioxy-phényl)-pyrazino[2',1':6,1]pyrido[3,4-b]indole-1,4-dione (TADALAFIL) ou un sel pharmaceutiquement acceptable de celle-ci.

16. Composition pharmaceutique selon l'une quelconque des revendications 9 à 11, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) ou un sel pharmaceutiquement acceptable de celle-ci.

17. Composition pharmaceutique selon l'une quelconque des revendications 9 à 11, **caractérisée en ce que** l'inhibiteur de la PDE4 est le 3-cyclopropylméthoxy-4-difluorométhoxy-N-(3,5-dichloropyrid-4-yl)benzamide (Roflumilast) ou un sel pharmaceutiquement acceptable de celui-ci et l'inhibiteur de la PDE5 est la 5-[2-éthoxy-5-(4-méthyl-1-pipérazinylsulfonyl)phényl]-1-méthyl-3-n-propyl-1,6-dihydro-7H-pyrazolo[4,3-d]pyrimidin-7-one (SILDENAFIL) ou un sel pharmaceutiquement acceptable de celle-ci.

18. Composition pharmaceutique selon l'une quelconque des revendications 12 à 14, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est la (6R,12aR)-2,3,6,7,12,12a-hexahydro-2-méthyl-6-(3,4-méthylènedioxy-phényl)-pyrazino[2',1':6,1]pyrido[3,4-b]indole-1,4-dione (TADALAFIL) ou un sel pharmaceutiquement acceptable de celle-ci.

19. Composition pharmaceutique selon l'une quelconque des revendications 12 à 14, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est la 2-[2-éthoxy-5-(4-éthylpipérazin-1-ylsulfonyl)phényl]-5-méthyl-7-propylimidazo[5,1-f][1,2,4]triazin-4(3H)one (VARDENAFIL) ou un sel pharmaceutiquement acceptable de celle-ci.

20. Composition pharmaceutique selon l'une quelconque des revendications 12 à 14, **caractérisée en ce que** l'inhibiteur de la PDE4 est le 3-cyclopropylméthoxy-4-difluorométhoxy-N-(3,5-dichloro-1-oxy-pyrid-4-yl)benzamide (Roflumilast-N-oxyde) ou un sel pharmaceutiquement acceptable de celui-ci et l'inhibiteur de la PDE5 est la 5-[2-éthoxy-5-(4-méthyl-1-pipérazinyl-sulfonyl)phényl]-1-méthyl-3-n-propyl-1,6-dihydro-7H-pyrazolo[4,3-d]pyrimidin-7-one (SILDENAFIL) ou un sel pharmaceutiquement acceptable de celle-ci.
